# EUROPEAN PATENT APPLICATION

(11) **EP 3 875 466 A1**
(43) Date of publication of application: **08.09.2021**
(21) Application number: 20201886.7
(22) Date of filing: 14.10.2020
(51) Int. Cl.: C07K 7/06

(54) **PROCESS FOR THE SYNTHESIS OF ETELCALCETIDE**

(30) Priority: 05.03.2020 EP 20161312; 25.03.2020 EP 20165444
(71) Applicant: Fresenius Kabi iPSUM S.r.l., 20060 Cassina de' Pecchi - Milano (IT); Alma Mater Studiorum University of Bologna, 40126 Bologna (IT)
(72) Inventor: MARTELLI, Giulia, 40126 Bologna (IT); CABRI, Walter, 40138 Bologna (IT); FERRAZZANO, Lucia, 40126 Bologna (IT); VIOLA, Angelo, 20060 Cassina de' Pecchi - MILANO (IT); TOLOMELLI, Alessandra, 40126 Bologna (IT); RICCI, Antonio, 20060 Cassina de' Pecchi - MILANO (IT)
(74) Representative: Fresenius Kabi Deutschland GmbH

(57) **Abstract**

The present invention provides a manufacturing process for etelcalcetide through direct disulfide bond formation with N-chlorosuccinimide in high yield and high purity.

## Description

### FIELD OF THE INVENTION

The present invention relates to a manufacturing process for a peptide, in particular for etelcalcetide and its related intermediates.

### BACKGROUND OF THE INVENTION

Etelcalcetide (SEQ ID NO: 1), formerly known as velcalcetide, is a calcimimetic peptide drug for the treatment of secondary hyperparathyroidism in patients undergoing hemodialysis. It is administered intravenously at the end of each dialysis session. Etelcalcetide functions by binding to and activating the calcium-sensing receptor in the parathyroid gland.

Etelcalcetide (I) is a N-terminal acetylated heptapeptide comprising a further cysteine linked through disulfide bond to the D-cysteine in position 1, and is represented by formula (I):

1                                                    5
Ac-D-Cys(L-Cys-OH)-D-Ala-D-Arg-D-Arg-D-Arg-D-Ala-D-Arg-NH2                    I

Etelcalcetide is also indicated as:
Ac*-c*(C)*arrrar-*NH₂

Many preparations have been described both in solution (LPPS) and in solid phase (SPPS), involving disulfide bond formation through a diverse range of reagents.

CN106928321 disclosed a preparation of etelcalcetide wherein the pre-formed and isolated Boc-L-Cys(SCI)-OtBu is reacted with the deprotected acetylated heptapeptide precursor (1-7) to form the disulfide bond in solution phase.

### SUMMARY OF THE INVENTION

Our inventors found that it is possible to couple L-cysteine with a heptapeptide precursor of etelcalcetide by direct treatment of the reactants with N-chlorosuccinimide (NCS), both in solution and in solid phase, without the need to pre-form and isolate the reactive species.

While it was expected that homo-dimerization would occur at least to some extent, i.e. that a first molecule of an heptapeptide (II), for instance (IIa)

Ac-D-Cys-D-Ala-D-Arg-D-Arg-D-Arg-D-Ala-D-Arg-NH₂ IIa

would react with a second molecule (IIa), resulting in the homodimer (IIIa) 
Ac-D-Cys-D-Ala-D-Arg-D-Arg-D-Arg-D-Ala-D-Arg-NH 2
S
S
IIIa
Ac-D-Cys-D-Ala-D-Arg-D-Arg-D-Arg-D-Ala-D-Arg-NH 2
 as an unwanted side product, it was surprisingly found that when reacting the reactants in the presence of NCS only the desired hetero-dimer product, etelcalcetide, was obtained. No side product was detected. The desired compound etelcalcetide was obtained in high purity when performed according to the process of the present invention.

Such method provides an efficient, simple and viable process which allows to obtain etelcalcetide with high yield and purity and has an important industrial application.

### DESCRIPTION

The present invention provides a process for the preparation of etelcalcetide (I) 
1                                   5
Ac-D-Cys(L-Cys-OH)-D-Ala-D-Arg-D-Arg-D-Arg-D-Ala-D-Arg-NH2          I
 comprising the step of coupling heptapeptide (II, SEQ ID NO: 2)

Ac-D-Cys-D-Ala-D-Arg(P_{A})-D-Arg(P_{A})-D-Arg(P_{A})-D-Ala-D-Arg(P_{A})-X II

with P-L-Cys-OH in the presence of N-chlorosuccinimide (NCS),
wherein X is NH₂ or a solid support,
P_{A} is hydrogen or a suitable protecting group for any arginine side chain and
P is hydrogen or an alpha-amino protecting group.

In one embodiment, X is NH₂, P_{A} and P are hydrogen.

In a second embodiment, X is a solid support, P is an alpha-amino protecting group and P_{A} is a protecting group for the arginine side chain, preferably Pbf. In this embodiment P is a protecting group cleavable in acidic conditions; preferably, P is Boc.

The heptapeptide (II)

Ac-D-Cys-D-Ala-D-Arg(P_{A})-D-Arg(P_{A})-D-Arg(P_{A})-D-Ala-D-Arg(P_{A})-X II

can be either protected at amino acids side-chains or not, that is, P_{A} is either a suitable side-chain protecting group or hydrogen.

Suitable side-chain protecting groups P_{A} may be any protecting group suitable for protection of the side-chain guanidino group of Arg residues, such as for example Pmc (2,2,5,7,8-pentamethylchroman-6-sulfonyl), bis-Boc, MIS (1,2-dimethylindole-3-sulfonyl) or Pbf (2,2,4,6,7-pentamethyl-dihydrobenzofuran-5-sulfonyl). Other protective groups for arginine side-chain are ω-5-dibenzosuberenyl (Suben), 5-dibenzosuberyl (Sub), and 2-methoxy-5-dibenzosuberyl (MeSub). The several Arg residues may be protected with the same P_{A} group or with different ones. It is one preferred embodiment wherein they are all the same, and wherein that P_{A} is Pbf.

In a preferred embodiment, the heptapeptide (II) is

Ac-D-Cys-D-Ala-D-Arg-D-Arg-D-Arg-D-Ala-D-Arg-NH₂ IIa

wherein no side-chain protecting groups are present, i.e. in heptapeptide of formula (II) P_{A} is hydrogen and X is NH₂.

In another preferred embodiment, the heptapeptide (II) is

Ac-D-Cys-D-Ala-D-Arg(Pbf)-D-Arg(Pbf)-D-Arg(Pbf)-D-Ala-D-Arg(Pbf)-X IIb,

wherein X is a solid support, all Arg residues are protected and no side-chain protecting group is present at the D-Cys, if not indicated otherwise.

In a preferred embodiment, the heptapeptide (II)

Ac-D-Cys-D-Ala-D-Arg(P_{A})-D-Arg(P_{A})-D-Arg(P_{A})-D-Ala-D-Arg(P_{A})-X

is prepared through Solid Phase Peptide Synthesis (SPPS) by stepwise elongation of the peptide sequence attached to a solid support, or resin, X. Alternatively, heptapeptide (II) is prepared through SPPS by condensation of shorter fragments.

In a preferred aspect of the present invention, solid support X is selected from the group comprising Rink amide, Rink amide AM and Rink amide MBHA resins. Such resins have the advantage that they allow obtaining directly a C-terminal amide after cleavage of the peptide from the resin, therefore they are particularly suitable for the preparation of etelcalcetide.

More preferably, in the process of the present invention Rink amide resin is used.

In another preferred aspect of present invention, the SPPS preparation of the heptapeptide is carried out by using the following protected amino acids as building blocks:
Fmoc-D-Arg(P_{A})-OH, for example Fmoc-D-Arg(Pmc)-OH,
Fmoc-D-Arg(Boc)₂-OH, Fmoc-D-Arg(MIS)-OH, or Fmoc-D-Arg(Pbf)-OH, wherein preferably Fmoc-D-Arg(Pbf)-OH is used,
Fmoc-D-Ala-OH, and
Fmoc-D-Cys(Trt)-OH or Fmoc-D-Cys(Mmt)-OH or Ac-D-Cys(Trt)-OH or Ac-D-Cys(Mmt)-OH.

When Fmoc-D-Cys(Trt)-OH or Fmoc-D-Cys(Mmt)-OH are incorporated into the intermediate heptapeptide, the intermediate heptapeptide is further acetylated after Fmoc removal, for instance by using AC₂O or another acetylating agent, preferably by using AC₂O in DMF.

Trt (trityl) and Mmt (monomethoxytrityl) are examples of suitable protecting groups for the side-chain sulfhydryl group of cysteine, which are cleavable in acidic conditions. Other protecting groups may be also suitable if they are cleaved in acidic conditions.

All coupling reactions taking place during the SPPS as described above are carried out under conditions that are well known by the person skilled in the art, preferably involving at least one coupling reagent and optionally an additive.

The coupling reagent may be selected from the group consisting of N-hydroxysuccinimide (NHS), N,N'-diisopropylcarbodiimide (DIC), N,N'-dicyclohexylcarbodiimide (DCC), (Benzotriazol-1-yloxy)tripyrrolidinophosphonium hexafluorophosphate (PyBOP), 2-(7-Aza-1H-benzotriazole-1-yl)-1,1,3,3-tetramethyluronium hexafluorophosphate (HATU), 2-(1H-benzotriazole-1-yl)-1,1,3,3-tetramethyluronium hexafluorophosphate (HBTU) and ethyl-dimethylaminopropyl carbodiimide (EDC). More preferably, the reaction is carried out in the presence of N,N'-diisopropylcarbodiimide (DIC).

The additive may be selected from the group consisting of 1-hydroxybenzotriazole (HOBt), 2-hydroxypyridine N-oxide, N-hydroxysuccinimide (NHS), 1-hydroxy-7-azabenzotriazole (HOAt), endo-N-hydroxy-5-norbornene-2,3-dicarboxamide, 5-(Hydroxyimino)1,3-dimethylpyrimidine-2,4,6-(1H,3H,5H)-trione (Oxyma-B) and ethyl 2-cyano-2-hydroxyimino-acetate (OxymaPure). More preferably, the additive is selected from the group consisting of 1-hydroxybenzotriazole (HOBt), 2-hydroxypyridine N-oxide, N-hydroxysuccinimide (NHS), 1-hydroxy-7-azabenzotriazole (HOAt), endo-N-hydroxy-5-norbornene-2,3-dicarboxamide, and ethyl 2-cyano-2-hydroxyimino- acetate (OxymaPure). Even more preferably, the reaction is carried out in the presence of 2-cyano-2-hydroxyimino-acetate (OxymaPure).

Optionally, an additional step to block unreacted sites is performed after each coupling step, often referred to as "capping", which shall avoid truncated sequences and shall prevent any side reactions. Capping is achieved by a short treatment of the loaded resin with a large excess of a highly reactive unhindered reagent. In the process of the present invention, capping is preferably performed by treatment with an acid derivative, such as an anhydride, for instance with a DMF/AC₂O solution.

Fmoc group removal is performed in basic conditions, for instance by treatment with a 20% piperidine solution in DMF.

In another embodiment, the heptapeptide (II)wherein X is NH₂ and P_{A} is not hydrogen, which is

Ac-D-Cys-D-Ala-D-Arg(P_{A})-D-Arg(P_{A})-D-Arg(P_{A})-D-Ala-D-Arg(P_{A})-NH₂ IIc

or the heptapeptide (IIa)

Ac-D-Cys-D-Ala-D-Arg-D-Arg-D-Arg-D-Ala-D-Arg-NH₂ IIa

is prepared through LPPS by condensation of shorter fragments obtained by repetition of single amide bonds formation, and optional final cleavage of protecting groups P_{A}, by using methods pertaining to peptide synthesis in liquid phase and known to the person skilled in the art.

The disulfide bond formation reaction is carried out with N-chlorosuccinimide (NCS) in a suitable solvent and at a suitable temperature. Suitable solvents comprise, for instance, water, acetonitrile (ACN), DMF and mixtures thereof. Preferably, the reaction is carried out at room temperature, i.e in the range 15-30°C. Preferably N-chlorosuccinimide is used in an amount of from 1 to 8 equivalents relative to heptapeptide (II), for instance with 6 eq overall. Preferably, NCS is added stepwise (repeatedly), until reaction completion.

In one embodiment, when the disulfide bond formation is carried out in solution phase, an intermediate protected heptapeptide (IVa, SEQ ID NO: 3) is prepared:

Ac-D-Cys(Trt)-D-Ala-D-Arg(Pbf)-D-Arg(Pbf)-D-Arg(Pbf)-D-Ala-D-Arg(Pbf)-X IVa

which is cleaved from the solid support X by means of an acidic cleaving mixture. For instance, a mixture of TFA/H₂O/TIPS/DODT 92.5/2.5/2.5/2.5 v/v/v/v, or a mixture of TFA/H₂O/TIPS 92.5/2.5/2.5 v/v/v, or a mixture TFA/H₂O/TI PS/dodecanthiol 90/5/2.5/2.5 v/v/v/v, or the like, can be used. Simultaneously, side-chain protecting groups are cleaved, providing heptapeptide IIa.

Said heptapeptide

Ac-D-Cys-D-Ala-D-Arg-D-Arg-D-Arg-D-Ala-D-Arg-NH₂ IIa

is then reacted with H-L-Cys-OH and NCS to provide etelcalcetide (I).

Preferably, such disulfide bond formation is carried out in an aqueous solvent mixture. More preferably, such mixture comprises acetonitrile. Most preferably, the aqueous solvent mixture is a mixture of water and acetonitrile, like for example, a mixture H₂O/CH₃CN 2:1. H-L-Cys-OH is generally used in excess, i.e. in an amount exceeding the stoichiometric amount. Preferably H-L-Cys-OH is used in an amount of from 1 to 10 equivalents relative to heptapeptide (IIa), more preferably from 5 to 10 eq, for instance with 8 eq overall. H-L-Cys-OH is preferably added to the reaction mixture stepwise or at intervals or repeatedly, together with or followed by the addition of NCS, until reaction completion.

In a second embodiment, when the disulfide bond formation is carried out while the heptapeptide II is still attached to the solid support X, an intermediate protected heptapeptide is prepared through SPPS as described above, for example heptapeptide (IVb), wherein P_{A} is Pbf:

Ac-D-Cys(Mmt)-D-Ala-D-Arg(Pbf)-D-Arg(Pbf)-D-Arg(Pbf)-D-Ala-D-Arg(Pbf)-X IVb.

In alternative embodiments the protective group at the Arg side-chains may be an alternative suitable protective group, as mentioned above.

Fmoc-D-Cys(Mmt)-OH is preferably employed as building block in this embodiment.

Then, the D-Cys side-chain protecting group (Mmt) is cleaved. The Mmt group can be cleaved in mild acidic conditions, for instance with 2% TFA in DCM.

The resulting heptapeptide IIb, as defined above, is reacted with P-L-Cys-OH and NCS, wherein P is as defined above. P-L-Cys-OH is preferably Boc-L-Cys-OH. Preferably, such disulfide bond formation is carried out in DMF.

The compound resulting from this disulfide bond formation, having following formula V:

Ac-D-Cys(L-Cys-OH)-D-Ala-D-Arg(Pbf)-D-Arg(Pbf)-D-Arg(Pbf)-D-Ala-D-Arg(Pbf)-X V

is then simultaneously cleaved from the solid support and deprotected at arginine residues side-chains, so that etelcalcetide (I) is obtained.

The crude target peptide etelcalcetide obtained by either embodiment of the present invention is optionally purified to further increase its purity. To this aim, a solution of the peptide is loaded onto an HPLC column with a suitable stationary phase, preferably C18 or C8 modified silica, and an aqueous mobile phase comprising an organic solvent, preferably acetonitrile or methanol, is passed through the column. A gradient of the mobile phase is applied, if necessary. The peptide with desired purity is collected and optionally lyophilized.

### ABBREVIATIONS

- SPPS: Solid Phase Peptide Synthesis
- LPPS: Liquid Phase Peptide Synthesis
- Fmoc Rink amide resin: 4-(2',4'-Dimethoxyphenyl-Fmoc-aminomethyl)-phenoxyacetamidomethyl polystyrene resin
- MBHA resin: Methyl benzhydryl amide resin
- Fmoc Rink amide MBHA resin: 4-(2',4'-Dimethoxyphenyl-Fmoc-aminomethyl)-phenoxyacetamido-4-methylbenzhydrylamine polystyrene resin
- Fmoc Rink amide AM resin: 4-(2',4'-Dimethoxyphenyl-Fmoc-aminomethyl)-phenoxyacetamido-aminomethyl resin
- Fmoc: 9-Fluorenylmethoxycarbonyl
- Boc: Tert-butyloxycarbonyl
- Pbf: 2,2,4,6,7-Pentamethyl-dihydrobenzofuran-5-sulfonyl
- Trt: Trityl
- Mmt: Monomethoxytrityl
- eq.: Equivalent
- h: hour/s
- min: minute/s
- rt: room temperature
- HPLC: High Performance Liquid Chromatography
- HPLC-MS: High Performance Liquid Chromatography coupled with Mass Spectrometry
- DIEA/DIPEA: N,N-Diisopropylethylamine
- TFA: Trifluoroacetic acid
- DODT: 3,6-dioxa-1,8-octanedithiol
- AC₂O: Acetic anhydride
- EtOAc: Ethyl acetate
- DMF: N,N-Dimethylformamide
- DMA: N,N-Dimethylacetamide
- DCM: Dichloromethane
- THF: Tetrahydrofuran
- NMP: N-Methyl-2-pyrrolidinone
- MTBE: Methyl-tert-butylether
- DIPE: Diisopropylether
- MeOH: Methanol
- HFIP: Hexafluoro-2-propanol
- TBDMS: Tert-butyl-dimethyl-silyl
- TIPS: Triisopropylsilane
- DIC: N,N'-Diisopropylcarbodiimide
- DCC: N,N'-Dicyclohexylcarbodiimide
- EDC: N-(3-dimethylaminopropyl)-N'-ethylcarbodiimide
- HOBt: 1-Hydroxybenzotriazole
- HOAt: 1-Hydroxy-7-azabenzotriazole
- NHS: N-Hydroxysuccinimide
- NCS: N-chlorosuccinimide
- TBTU: O-(Benzotriazol-1-yl)-N,N,N',N'-tetramethyluronium tetrafluoroborate
- HBTU: O-(Benzotriazol-1-yl)-N,N,N,N-tetramethyluronium hexafluorophosphate
- HATU: 2-(7-Aza-1H-benzotriazole-1-yl)-1,1,3,3-tetramethyluronium hexafluorophosphate
- PyBOP: (Benzotriazol-1-yloxy)tripyrrolidinophosphonium hexafluorophosphate
- OxymaPure: Ethyl 2-cyano-2-hydroxyimino-acetate
- Pmc: 2,2,5,7,8-Pentamethylchroman-6-sulfonyl
- Bis-Boc: (BOC)₂
- MIS: 1,2-Dimethylindole-3-sulfonyl

### EXPERIMENTAL PART

### Preparation of heptapeptide (IVa)

SPPS of heptapeptide Ac-D-Cys(Trt)-D-Ala-D-Arg(Pbf)-D-Arg(Pbf)-D-Arg(Pbf)-D-Ala-D-Arg(Pbf)-X was conducted by means of standard Fmoc chemistry using:
Resin (X): Rink Amide MBHA (Loading: 0.8 mmol/g, 10 g)
Deprotection: 20% piperidine solution in DMF (2 x 60 mL x 10 min)
Washings: DMF (3 x 60 mL)
Couplings: Fmoc-AA-OH (2 eq., 16 mmol), OxymaPure (2 eq., 16 mmol, 2.3 g) in DMF (32 mL), DIC (2 eq., 16 mmol, 2.5 mL), preactivation for 3 min, then addition to the resin (75 min, with an optional second cycle)
Employed amino acids: Fmoc-D-Arg(Pbf)-OH (10.4 g), Fmoc-D-Ala-OH (5.0 g), and Fmoc-D-Cys(Trt)-OH (9.4 g) or Ac-D-Cys(Trt)-OH (6.5 g). When Fmoc-D-Cys(Trt)-OH was used, the intermediate heptapeptide was further acetylated after Fmoc removal, by using AC₂O in DMF.
Capping: AC₂O (1.84 mL) in DMF (58.16 mL)

### Preparation of heptapeptide Ac-D-Cys-D-Ala-D-Ara-D-Ara-D-Ara-D-Ala-D-Ara-NH₂ (IIa)

The intermediate protected heptapeptide IVa prepared as reported above was cleaved from the solid support by means of an acidic cleaving mixture, with simultaneous cleavage of the side-chain protecting groups.

The dry resin was added portionwise to the cleavage mixture TFA 90%, H₂O 5%, TIPS 2.5%, dodecanthiol 2.5% (200 mL, 20 mL cleavage mixture/1 g resin) and stirred for 2 hours. The resin was filtered, washed once with TFA (40 mL), then filtered again. The filtrate was cooled to 0°C, then DIPE (500 mL) was added dropwise under stirring. The solid precipitate was filtered, washed with DIPE (50 mL) and dried under vacuum for 48h to obtain the title compound (7.3 g, 7.9 mmol, purity 97.2%), which was further used without purification.

### Preparation of etelcalcetide (I): Disulfide bond formation in solution phase

7.3 g of heptapeptide IIa from the previous step were diluted in a mixture of H₂O/CH₃CN 2:1 (360 mL) under stirring. H-L-Cys-OH (2 eq, 15.8 mmol, 1.9 g) was added, followed by addition of NCS (1.5 eq, 1.6 g) at rt. After 1 h, a second addition of H-L-Cys-OH (2 eq, 1.9 g) and NCS (1.5 eq, 1.6 g) was performed. Other two further additions were performed every 60 minutes, then the mixture was stirred for 16h at rt. HPLC monitoring showed complete conversion of heptapeptide IIa to etelcalcetide I after 4 total additions of H-L-Cys-OH (8 eq, 7.6 g) and NCS (6 eq, 6.4 g).

At completion, the reaction mixture comprising the dissolved crude target peptide was filtered, the solid discarded and the filtrate was straight injected in preparative HPLC without any work-up. Selected fractions were lyophilized after freeze-drying and 7.8 g etelcalcetide were obtained with a final purity of 99.1% (yield 94%).

### Chromatographic details:

Preparative HPLC: Agilent 1100, column: Phenomenex Luna® C18 (250 x 21.2 mm), flow: 10 mL/min, temperature: 25°C; UV: 210 nm, eluent: H₂O+0.1%TFA/CH₃OH+0.1%TFA 90:10.

Analytical HPLC-MS: Agilent 1260 Infinity II system coupled to an electrospray ionization mass spectrometer (positive-ion mode, m/z = 100-1500, fragmentor 30 V).
1) column: Poroshell120 EC-C18 2.7 µm, 100 x 4.6 mm; flow: 0.5 mL/min, temperature: 25°C; injection volume: 30 µL, UV: 215 nm, elution phases: H₂O+0.1%TFA (A) /CH₃OH+0.1%TFA (B) - gradient 5% to 95% (B) in 50 min.
2) column: Zorbax-SB-C18 5 µm, 250 x 4.6 mm; flow: 0.5 mL/min, temperature: 25°C; injection volume: 30 µL, UV: 220 nm, elution phase: H₂O+0.08%TFA (FMA) /CH₃CN+0.08%TFA (FMB) - gradient 0% to 60% (B) in 30 min.

## Claims

1. A process for the preparation of etelcalcetide (I, SEQ ID NO: 1)) 
1                                                    5
Ac-D-Cys(L-Cys-OH)-D-Ala-D-Arg-D-Arg-D-Arg-D-Ala-D-Arg-NH2                    I
 comprising the step of coupling heptapeptide (II, SEQ ID NO: 2)
Ac-D-Cys-D-Ala-D-Arg(P_{A})-D-Arg(P_{A})-D-Arg(P_{A})-D-Ala-D-Arg(P_{A})-X II
with P-L-Cys-OH in the presence of N-chlorosuccinimide,
wherein X is NH₂ or a solid support,
and wherein if X is NH₂, P is hydrogen, and P_{A} is hydrogen,
and wherein if X is a solid support, P is a protecting group and P_{A} is a protecting group suitable for arginine residues.

2. The process according to claim 1, wherein X is a solid support and P is a protecting group and P_{A} is a protecting group suitable for arginine residues.

3. The process according to claim 2, wherein P is Boc.

4. The process according to claims 2 or 3, wherein X is Rink amide resin, or another solid support selected from the group consisting of Rink amide AM and Rink amide MBHA resin.

5. The process according to any one of claims 2 to 4, wherein
Ac-D-Cys-D-Ala-D-Arg(P_{A})-D-Arg(P_{A})-D-Arg(P_{A})-D-Ala-D-Arg(P_{A})-X (II)
is prepared through stepwise SPPS using Fmoc-D-Cys(Mmt)-OH or Ac-D-Cys(Mmt)-OH.

6. The process according to any one of claims 2 to 5, wherein P_{A} is selected from the group consisting of Pbf, Pmc, bisBoc and MIS.

7. The process according to claim 6, wherein P_{A} is Pbf.

8. The process according to any one of claims 2 to 6, wherein the coupling is performed in DMF.

9. The process according to claim 1, wherein X is NH₂ and P is hydrogen.

10. The process according to claim 9, wherein the coupling is performed in an aqueous solvent mixture.

11. The process according to claim 10, wherein the aqueous solvent mixture comprises acetonitrile.

12. The process according to claim 10 or 11, wherein the aqueous solvent mixture is a mixture of water and acetonitrile.

13. The process according to any of claims 9 to 12, wherein heptapeptide (II) corresponds to
Ac-D-Cys-D-Ala-D-Arg-D-Arg-D-Arg-D-Ala-D-Arg-NH₂ IIa
and is prepared through stepwise or fragment-based SPPS on a solid support, and wherein its precursor of formula (IVa, SEQ ID NO: 3)
Ac-D-Cys(Trt)-D-Ala-D-Arg(Pbf)-D-Arg(Pbf)-D-Arg(Pbf)-D-Ala-D-Arg(Pbf)-X IVa
wherein X is a solid support,
is cleaved from the solid support before the coupling with H-L-Cys-OH in the presence of N-chlorosuccinimide.

14. The process according to any of claims 9 to 13, wherein heptapeptide (II) corresponds to
Ac-D-Cys-D-Ala-D-Arg-D-Arg-D-Arg-D-Ala-D-Arg-NH₂ IIa
and is prepared through stepwise or fragment-based SPPS on a solid support and Fmoc-D-Cys(Trt)-OH or Ac-D-Cys(Trt)-OH is used during said preparation.

15. The process according to any of the preceding claims, wherein N-chlorosuccinimide is used in an amount of from 1 to 8 molar equivalents relative to the heptapeptide (II).


SEQUENCE LISTING

<110>  Fresenius Kabi iPSUM S.r.l.
       Alma mater Studiorum - University of Bologna

<120>  PROCESS FOR THE SYNTHESIS OF ETELCALCETIDE

<130>  FK19302-05-PAT-EP

<160>  3

<170>  PatentIn version 3.5

<210>  1
<211>  7
<212>  PRT
<213>  Artificial Sequence

<220>
<223>  Etelcalcetide



<220>
<221>  VARIANT
<222>  (1)..(1)
<223>  Xaa = D-Cys

<220>
<221>  MOD_RES
<222>  (1)..(1)
<223>  ACETYLATION N-terminal

<220>
<221>  DISULFID
<222>  (1)..(1)
<223>  disulfide bond with L-Cys

<220>
<221>  VARIANT
<222>  (2)..(2)
<223>  Xaa = D-Ala

<220>
<221>  VARIANT
<222>  (3)..(3)
<223>  Xaa = D-Arg

<220>
<221>  VARIANT
<222>  (4)..(4)
<223>  Xaa = D-Arg

<220>
<221>  VARIANT
<222>  (5)..(5)
<223>  Xaa = D-Arg

<220>
<221>  VARIANT
<222>  (6)..(6)
<223>  Xaa = D-Ala

<220>

<221>  VARIANT
<222>  (7)..(7)
<223>  Xaa = D-Arg

<220>
<221>  MOD_RES
<222>  (7)..(7)
<223>  AMIDATION C-terminal

<400>  1

Xaa Xaa Xaa Xaa Xaa Xaa Xaa
1               5


<210>  2
<211>  7
<212>  PRT
<213>  Artificial Sequence

<220>
<223>  heptapeptide II


<220>
<221>  VARIANT
<222>  (1)..(1)
<223>  Xaa = D-Cys

<220>
<221>  MOD_RES
<222>  (1)..(1)
<223>  ACETYLATION N-terminal

<220>
<221>  VARIANT
<222>  (2)..(2)
<223>  Xaa = D-Ala

<220>
<221>  VARIANT
<222>  (3)..(3)
<223>  Xaa = D-Arg, optionally protected at side-chain

<220>
<221>  VARIANT
<222>  (4)..(4)
<223>  Xaa = D-Arg, optionally protected at side-chain

<220>
<221>  VARIANT
<222>  (5)..(5)
<223>  Xaa = D-Arg, optionally protected at side-chain

<220>
<221>  VARIANT
<222>  (6)..(6)
<223>  Xaa = D-Ala

<220>
<221>  VARIANT
<222>  (7)..(7)
<223>  Xaa = D-Arg, optionally protected at side-chain

<220>
<221>  MOD_RES
<222>  (7)..(7)
<223>  AMIDATION C-terminal or attachment to solid support

<400>  2

Xaa Xaa Xaa Xaa Xaa Xaa Xaa
1               5


<210>  3
<211>  7
<212>  PRT
<213>  Artificial Sequence

<220>
<223>  heptapeptide IVa


<220>
<221>  VARIANT
<222>  (1)..(1)
<223>  Xaa = D-Cys protected with Trt at side-chain

<220>
<221>  MOD_RES
<222>  (1)..(1)
<223>  ACETYLATION N-terminal

<220>
<221>  VARIANT
<222>  (2)..(2)
<223>  Xaa = D-Ala

<220>
<221>  VARIANT
<222>  (3)..(3)
<223>  Xaa = D-Arg

<220>
<221>  VARIANT
<222>  (4)..(4)
<223>  Xaa = D-Arg protected with Pbf at side-chain

<220>
<221>  VARIANT
<222>  (5)..(5)
<223>  Xaa = D-Arg protected with Pbf at side-chain

<220>
<221>  VARIANT
<222>  (6)..(6)
<223>  Xaa = D-Ala

<220>
<221>  VARIANT
<222>  (7)..(7)
<223>  Xaa = D-Arg protected with Pbf at side-chain

<220>
<221>  MOD_RES

<222>  (7)..(7)
<223>  C-terminal attachment to solid support

<400>  3

Xaa Xaa Xaa Xaa Xaa Xaa Xaa
1               5